# EUROPEAN PATENT SPECIFICATION

(11) **EP 2 690 088 B1**
(45) Date of publication and mention of the grant of the patent: **22.02.2017**
(21) Application number: 12761142.4
(22) Date of filing: 22.03.2012
(51) Int. Cl.: C07C 233/10, C07C 231/24, A61K 31/165, A61P 25/20, A61P 25/22, A61P 25/00, A61P 9/00, A61P 1/00, A61P 25/24

(54) **MIXED CRYSTAL AGOMELATINE (FORM-VIII), PREPARATION METHOD AND USE THEREOF AND PHARMACEUTICAL COMPOSITION CONTAINING SAME**
MISCHKRISTALL-AGOMELATIN DER FORM VIII, HERSTELLUNGSVERFAHREN DAFÜR UND VERWENDUNG DAVON SOWIE PHARMAZEUTISCHE ZUSAMMENSETZUNG DAMIT
AGOMÉLATINE CRISTALLINE MIXTE (FORME VIII), SON PROCÉDÉ DE PRÉPARATION ET UTILISATION ET COMPOSITION PHARMACEUTIQUE LA CONTENANT

(30) Priority: 23.03.2011 CN 201110070634
(43) Date of publication of application: 29.01.2014
(73) Proprietor: Les Laboratoires Servier, 92284 Suresnes Cedex (FR); Shanghai Institute Of Pharmaceutical Industry, Shanghai 200040 (CN)
(72) Inventor: HUANG, Yu, Shanghai 200122 (CN); LONG, Qing, Shantou, Guangdong 515000 (CN); ZHU, Xueyan, Shanghai 200436 (CN); SHAN, Hanbin, Gaoan, Jiangxi 330800 (CN); YUAN, Zhedong, Shanghai 200060 (CN); YU, Xiong, Shanghai 200081 (CN)
(74) Representative: Bestel, Delphine
(86) International application number: PCT/CN2012/072818
(87) International publication number: WO 2012/126386

(56) References cited:
- WO-A1-2011/054917
- CN-A- 101 429 134
- CN-A- 101 585 779
- CN-A- 101 723 844
- CN-A- 101 723 844
- CN-A- 101 792 400
- CN-A- 101 955 440
- TINANT, B. ET AL.: 'N-[2-(7-Methoxy-1-naphthyl)ethyl]-acetamid e, a potent melatonin analog' ACTA CRYST. vol. C50, 1994, pages 907 - 910, XP055123493

## Description

### Technical field

The present invention relates to a mixed crystalline form of agomelatine, N-[2-(7-methoxy-1-naphthyl)ethyl]acetamide, its method of preparation, application and pharmaceutical composition.

### Prior art

Agomelatine, with chemical name N-[2-(7-methoxy-1-naphthyl)ethyl]acetamide and brand name Valdoxan, has the following chemical structure:

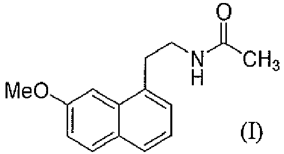

It has a dual effect, acting not only as an agonist of melatoninergic system receptors, but also as an antagonist of the 5HT_{2C} receptor. Its properties mean that it is active in the central nervous system, especially in the treatment of severe depression, seasonal affective disorder, sleep disorders, cardiovascular diseases, digestive system diseases, insomnia and fatigue brought on by jet lag, eating disorders and obesity. Agomelatine is the first melatoninergic antidepressant, and is effective in the treatment of depression and the improvement of sleep parameters, while not affecting sexual function.

The preparation and therapeutic use of agomelatine have been reported in the European patent EP0447285.

In view of the pharmaceutical value of said compound, it is important to obtain a highly pure, stable crystalline form with good reproducibility in order for it to be advantageous in pharmaceutical preparation and stable enough for long-term storage without having specific requirements in terms of temperature, light, humidity or oxygen levels.

The Chinese patents CN200510071611.6, CN200610108396.7, CN200610108394.8, CN200610108395.2, CN200910047329.2, CN200910245029.5 and CN101723844 have made public the various crystalline forms as well as the preparation methods of agomelatine.

Among these, in CN200910047329.2, the publicly disclosed crystalline form VI obtained through the process of acetic acid and water recrystallization has superior solubility over most of the existing publicly disclosed crystalline forms, thus possessing unique value with regard to its properties in pharmaceutical formulation. However, when placing crystalline form VI under extreme conditions (high temperature of 60°C) for 10 days, small amounts of crystal transition can occur.

Researchers have focused on the search for a crystalline or mixed crystalline form possessing greater stability without compromising in terms of solubility.

Advantageously, through exploring various preparation processes and comparisons of stability, the inventor has found a mixed crystalline form which, when placed under extreme conditions, offers superior stability over crystalline form VI. Said mixed crystal achieves greater stability without compromising the excellent solubility of crystalline form VI. It offers great reproducibility and under extreme conditions, stability in its preparation process, thus greatly increasing the feasibility of pharmaceutical formulation.

### Scope of the Invention

The aim of the present invention is to provide a mixed crystalline form of agomelatine, form VIII, while also providing a preparation process. When compared with crystalline form VI, said form VIII offers greater stability under high temperature. In addition, it exhibits valuable pharmaceutical formulation properties.

The mixed crystalline form VIII of agomelatine in the present invention may be used in the treatment of diseases of the melatoninergic system, sleep disorders, stress, anxiety, seasonal affective disorder, severe depression, cardiovascular diseases, digestive system diseases, insomnia and fatigue brought on by jet lag, schizophrenia, phobias, and depression.

The present invention also aims to provide a method of preparation for form VIII of agomelatine which is simple in its operation and offers good reproducibility.

A further aim of the present invention is to provide a pharmaceutical composition, which includes the mixed crystalline form VIII of agomelatine of this invention as well as pharmaceutically acceptable adjuvants or excipients.

The said pharmaceutical composition can be configured to be used in different application routes, especially when administered either orally or via injection. According to the nature and severity of the illness, treatment may be administered via a regulated dosage based on the age and weight of the patient. The dosage may vary between 0.1 mg and 1g per day, being administered once only or several times.

The following examples of X-ray diffraction diagrams of form VIII of agomelatine of the present invention use interplanar crystal spacing d, Bragg angle 2θ and relative intensity (I%) to show:

| 2θ° | d (Å) | Relative intensity (I%) |
|---|---|---|
| 9.493 | 9.3085 | 12.86 |
| 9.809 | 9.0096 | 15.62 |
| 10.815 | 8.1735 | 13.10 |
| 11.171 | 7.9141 | 17.53 |
| 11.879 | 7.4439 | 64.67 |
| 12.770 | 6.9264 | 17.90 |
| 13.811 | 6.4065 | 17.10 |
| 14.939 | 5.9255 | 12.14 |
| 15.315 | 5.7808 | 10.48 |
| 16.085 | 5.5057 | 19.89 |
| 17.544 | 5.0510 | 48.47 |
| 18.491 | 4.7943 | 66.41 |
| 19.065 | 4.6512 | 24.02 |
| 19.538 | 4.5398 | 99.39 |
| 19.774 | 4.4861 | 100.00 |
| 20.801 | 4.2668 | 50.35 |
| 21.156 | 4.1961 | 30.66 |
| 21.807 | 4.0722 | 37.31 |
| 22.499 | 3.9486 | 22.63 |
| 23.032 | 3.8583 | 31.18 |
| 23.780 | 3.7387 | 39.67 |
| 24.610 | 3.6144 | 21.02 |
| 25.419 | 3.5011 | 30.30 |
| 27.075 | 3.2906 | 14.67 |
| 31.931 | 2.8004 | 14.14 |

When using X-ray diffraction to measure the crystallization of the present invention, sometimes owing to the measurement equipment or test conditions, the measured peaks show slight deviations in measurement; more specifically, for example there may be a deviation in measurement of the 2θ value by approximately ±0.2; even if extremely accurate equipment is used, a deviation of approximately ±0.1 may be seen. As a result, this deviation must be taken into consideration when determining each crystalline structure.

XRD test conditions for said form VIII of agomelatine of the present invention: Instrument model: Bruker D8 ADVANCE X-ray diffractometer
Experiment parameters:
   Detector: LynxEye detector
   Light source: CuKα 40 kV 40 mA
   Monochromator: Ni filter disc
   Divergence slit: 1 °
   DivH.L.Slit: 1.0 mm
   Probe: LynxEye probe
   Scanning method: θ-θ continuous scanning
   Scanning range: 3°∼45°
   Step length: 0.02°
   Scanning speed: 8.0°/min
   Scanning time: 5 min
   Scanning temperature: Room temperature

Test conditions for DSC change-in-absorption diagram of said form VIII of agomelatine of the present invention:
Instrument model: NETZSCH DSC 204F1
Experimental conditions:
   Crucible type: Standard aluminium crucible (perforated)
   Shielding gas: High purity nitrogen 20 ml/min
   Sweep gas: High purity nitrogen 60 ml/min
   Heating rate: 10°C/min
   Temperature range: Room temperature ∼140°C

The onset value of the endothermic peak of the DSC change-in-absorption diagram of the present invention is characterised by: onset value range being 97-98°C, the endothermic peak area being no lower than 90%, with the preferable ratio being 95-99%.

When using DSC to measure the crystals of the present invention, sometimes owing to the measurement equipment or test conditions, the measured peaks show slight deviations in measurement; more specifically, for example there may be a deviation in measurement of the onset value by approximately ±1°C, even if extremely accurate equipment is used, a deviation of approximately ±0.5°C may be seen. As a result, this deviation must be taken into consideration when determining each crystalline structure.

TGA test conditions of the present invention:
Instrument model NETZSCH TG 209F1
Experimental conditions:
   Crucible type: Al₂O₃
   Sweep gas: N₂ 20 ml/min; shielding gas: N₂ 10 ml/min
   Temperature range: Room temperature∼300°C
   Heating rate: 10°C/min

The method of preparation of form VIII of the present invention involves dissolving agomelatine compound of formula (II) (Agomelatine-HCl-H₂O) in acetic acid, to which sodium acetate is then added, water is then added dropwise to this reaction mixture and agitated at a temperature of 7-13°C in order to bring about crystallization, with the crystals then being separated from the solution.

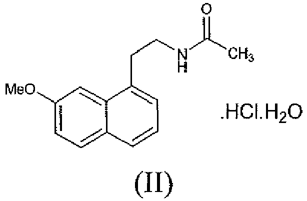

In the present invention as described, there are no special requirements in terms of the amount of acetic acid that is to be added as long as a sufficient amount is used to dissolve the raw materials, while heating can also be suitably applied to facilitate dissolution.

The molar ratio of agomelatine compound of formula (II) and sodium acetate is preferably of the order of 1:1-1.5, most optimally 1:1-1.1.

In the preparation method of the present invention as described, the ratio of volume of acetic acid to water is 1:15-30.

In a preferred embodiment of the preparation method for agomelatine form VIII in the present invention, when the temperature of the resulting reaction mixture reaches 12-18°C, and in particular when around 15°C, water is added dropwise in order to bring about crystallization.

In a further preferred embodiment, when water is added dropwise to the resulting reaction mixture, agitation is then carried out at a temperature of around 10°C. This may be carried out over a period of around 1.5 hours in order to bring about crystallization.

In another preferred embodiment, following the addition of sodium acetate, the reaction mixture is heated to 40-80°C, an appropriate, non-fixed, amount of activated carbon is then added, followed by agitation and filtration; said solution is then left to cool on its own, and water is added dropwise in order to bring about crystallization.

The agomelatine form VIII provided by the present invention can be used in conjunction with pharmaceutically acceptable adjuvants or excipients for pharmaceutical formulation.

The present invention results in a new form VIII of agomelatine, with greater stability compared to that of crystalline form VI, thus possessing advantages in production in terms of stability.

According to the Chinese patent application CN 201010126254.X, agomelatine compound of formula (II) as previously described may be produced by means of the following preparation method, where said preparation method involves reacting agomelatine with various forms of HCl in order to form a hydrate. The two methods are as follows: Agomelatine is firstly dissolved in a water-containing organic solvent, after which HCl gas is added, the solid crystals are washed and then dried; or else agomelatine is added to a solvent containing HCl, and the solid crystals are then washed and dried. If the first method is used, an overabundance of HCl may lead to a decrease in yield, while in the second method the amount of HCl present in the solvent is easily controlled. Therefore the second method is preferred.

Specifically, agomelatine may be added to a water-containing organic solvent, followed by the dropwise addition of a solvent containing HCl. The solid crystals are then washed and then dried.

Likewise, it is also possible to add agomelatine to an organic solvent, followed by the dropwise addition of an aqueous solution containing HCl. The solid crystals are then washed and then dried. The full contents of reference documents either quoted or mentioned in this application have been referenced.

### Description of drawings

Figure 1 shows the X-ray diffraction diagram of form VIII in embodiment 1 of the present invention;
Figure 2 shows the DSC change-in-absorption diagram of form VIII in embodiment 1 of the present invention;
Figure 3 shows the X-ray diffraction diagram of form VIII in embodiment 2 of the present invention;
Figure 4 shows the DSC change-in-absorption diagram of form VIII in embodiment 2 of the present invention;
Figure 5 shows the X-ray diffraction diagram of form VIII in embodiment 3 of the present invention;
Figure 6 shows the DSC change-in-absorption diagram of form VIII in embodiment 3 of the present invention;
Figure 7 shows the thermogravimetric analysis TGA curve of the product in embodiment 5 of the present invention.

### Details of the embodiments

The following embodiments further describe the present invention but do not limit the scope thereof.

### Embodiment 1:

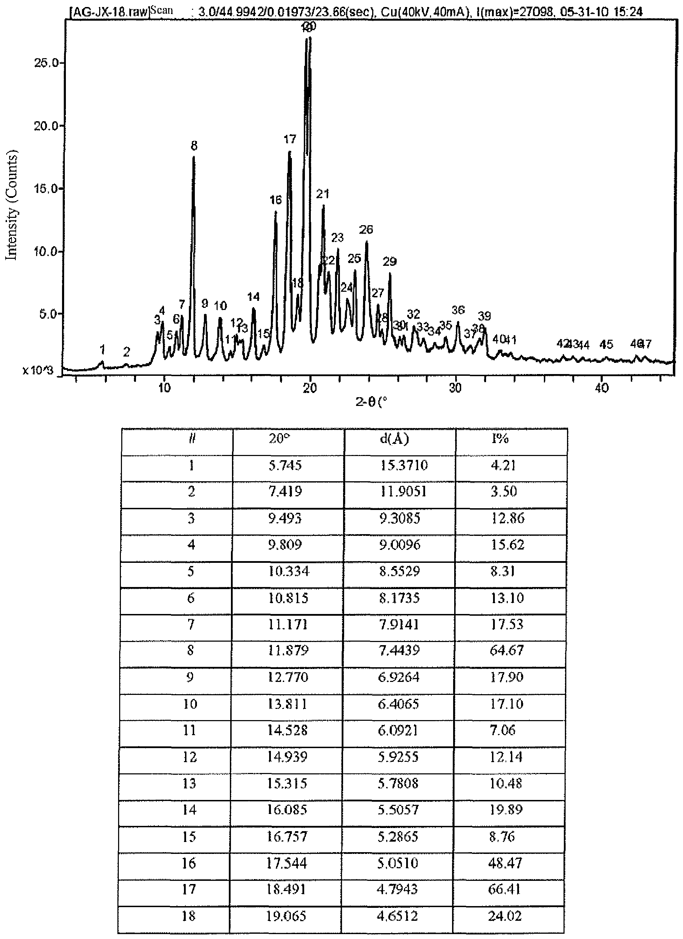

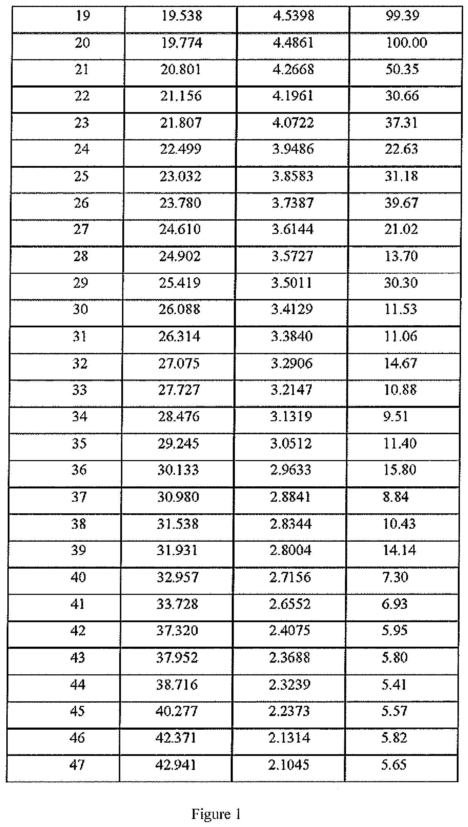

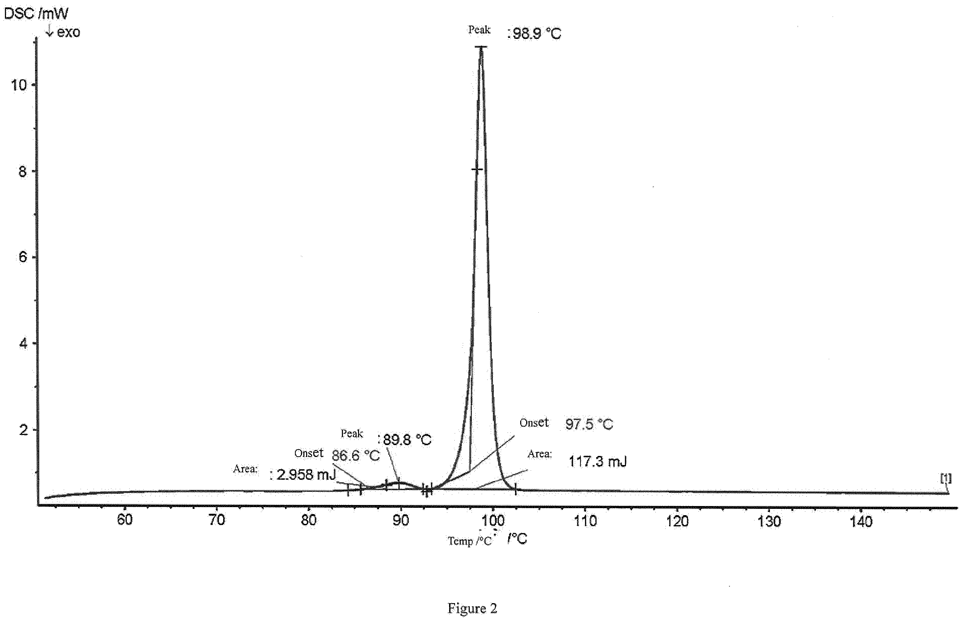

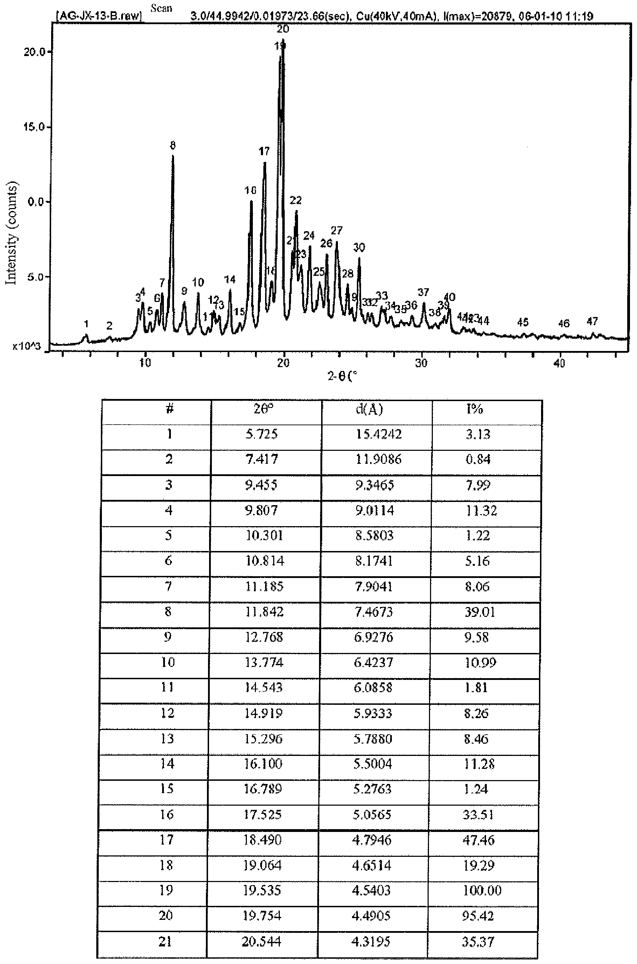

14g of agomelatine compound of formula (II) is dissolved in 55ml of acetic acid, to which 4.5g of sodium acetate is then added; the mixture is then heated to 60°C, after which 0.5g of activated carbon is added. Agitation is carried out for 2 hours after which the mixture is filtered; at a temperature of 15°C, 1L of water is then added dropwise. The solution gradually becomes turbid, and at a temperature of ∼10°C, agitation is carried out over 1.5 hours, followed by filtration, then washing and drying the filter cake at 45 °C under vacuum until constant weight is achieved, resulting in 9.6g of white solid;
(Refer to Figure 1 for X-ray diffraction diagram; refer to Figure 2 for DSC change-in-absorption diagram)

### Embodiment 2:

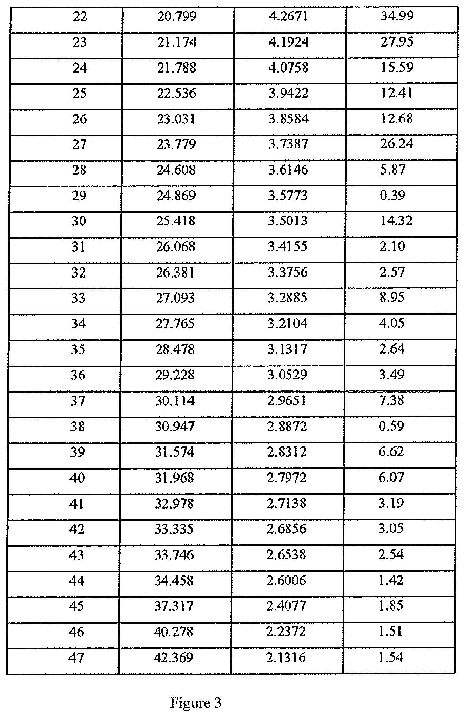

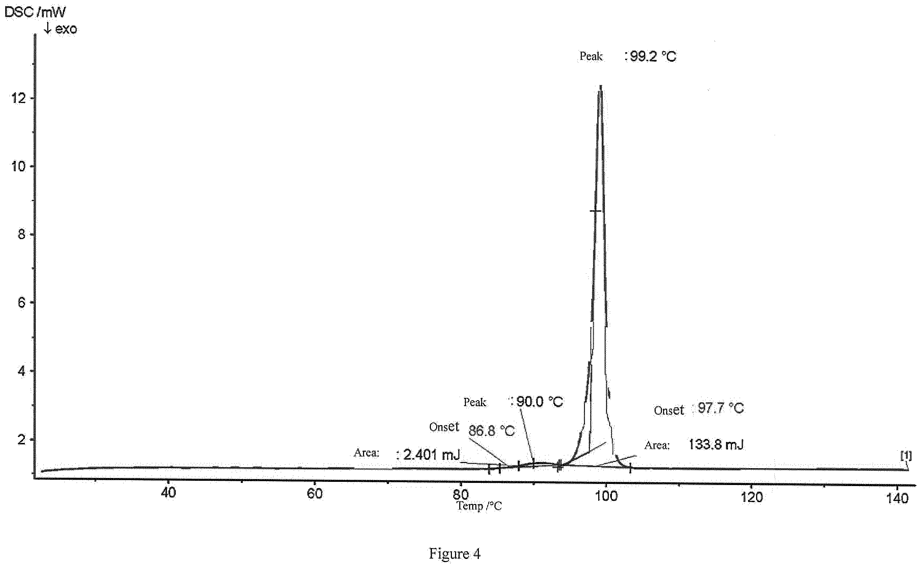

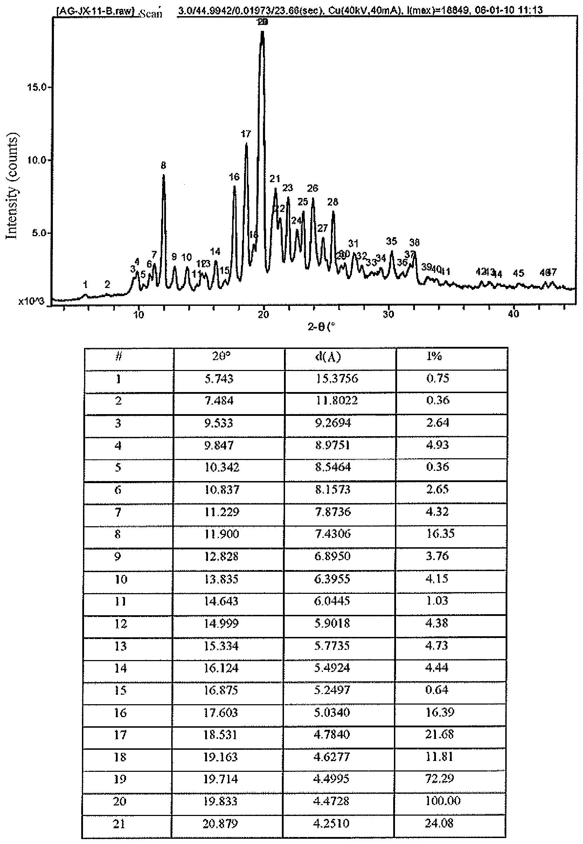

140g of agomelatine compound of formula (II) is dissolved in 490ml of acetic acid, to which 60g of sodium acetate is then added; the mixture is then heated to 60°C, after which 1.4g of activated carbon is added. Agitation is carried out for 1 hour after which the mixture is filtered; at a temperature of 15°C, 8.8L of water is then added dropwise. The solution gradually becomes turbid, and at a temperature of ∼10°C, agitation is carried out over 1.5 hours, followed by filtration, then washing and drying the filter cake at 45°C under vacuum until constant weight is achieved, resulting in 94g of white solid;
(Refer to Figure 3 for X-ray diffraction diagram; refer to Figure 4 for DSC change-in-absorption diagram)

### Embodiment 3:

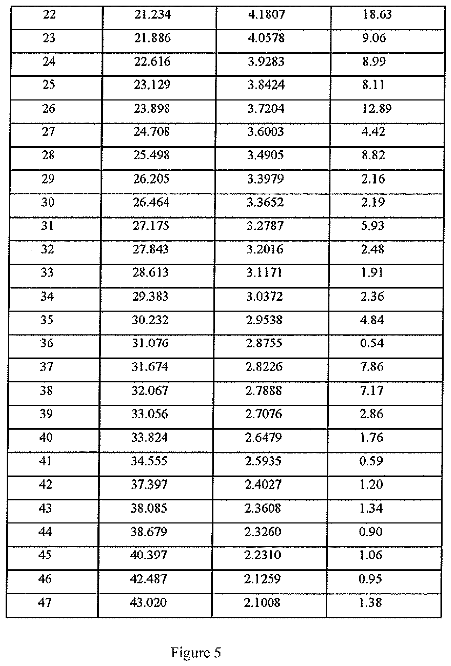

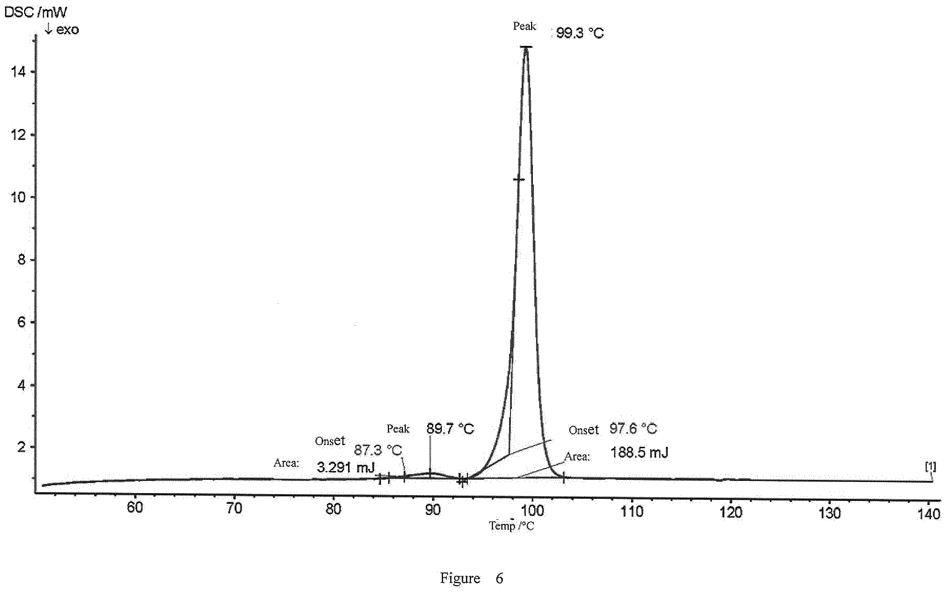

66g of agomelatine compound of formula (II) is dissolved in 230ml of acetic acid, to which 21g of sodium acetate is then added; the mixture is then heated to 60°C, after which 1.3g of activated carbon is added. Agitation is carried out for 1 hour after which the mixture is filtered; at a temperature of 15°C, 6.9L of water is then added dropwise. The solution gradually becomes turbid, and at a temperature of ∼10°C, agitation is carried out over 1.5 hours, followed by filtration, then washing and drying the filter cake at 50°C under vacuum until constant weight is achieved, resulting in 49g of white solid;
(Refer to Figure 5 for X-ray diffraction diagram; refer to Figure 6 for DSC change-in-absorption diagram)

### Embodiment 4:

Agomelatine crystalline forms VI and VIII (obtained through embodiment 2) are each placed in thermostatic containers at a temperature of 40°C and stored for 20 days, with the stability of these samples being studied using the method of High Performance Liquid Chromatography.

### 1. Purity measurement of the sample

Chromatographic conditions: Octadecyl silane chemically bonded silica is used as packing; a mixed solution of 10 mmol/L phosphate buffer (adjusted to pH 7.0 with sodium hydroxide) and acetonitrile in the ratio 2:7 by volume acts as the mobile phase; column temperature 40°C; and detection wavelength 220nm. Purity is measured using an internal standard method.

In the mobile phase, crystalline forms VI and VIII are distributed into 1mg/mL solutions, 10µL of each of which are then passed into a liquid chromatograph, with their chromatograms being recorded.

### 2. Assay of the Sample

The reference sample purity measurement method was used, with measurements being made using an external standard method, the results can be seen in Table I.

**Table I**

| Sample name | Crystalline form VI | | Form-VIII | |
|---|---|---|---|---|
| | Purity | Content | Purity | Content |
| Before storage | 99.7% | 100.1% | 99.8% | 100.3% |
| After storage in the thermostatically controlled containers for 20 days | 99.6% | 99.8% | 99.7% | 100.1% |

### 3. Measurement of water solubility

The HPLC method was used to determine water solubility, with measurements being made using an external standard method. The results are shown in Table II.

**Table II**

| Sample name | Crystalline form VI | Form-VIII |
|---|---|---|
| Solubility (mg/ml) | 0.336 | 0.335 |

### 4. Determination of crystalline stability

Measured using the pharmacopoeia stability assessment method:
1) Influencing factor testing (exposed for 10 days): High temperature (60°C), illumination (4500 1x), high humidity (92.5%RH, 25°C)
2) Accelerated testing (hermetically sealed for 6 months): Temperature 30°C, humidity 65%RH
3) Long term testing (hermetically sealed for 12 months): Temperature 25°C, humidity 60%RH

**Table III**

| Sample name | | Crystalline form VI | Form VIII |
|---|---|---|---|
| Influencing factor | High temperature | x* | √* |
| | Illumination | √ | √ |
| | High humidity | √ | √ |
| Accelerated testing | | √ | √ |
| Long term testing (6 months) | | √ | √ |
| Long term testing (9 months) | | √ | √ |
| Long term testing (12 months) | | x | √ |
| *: √- stable; ×- unstable | | | |

As can be seen from the test results, form VIII of agomelatine of the present invention clearly offers greater stability under high temperature and comparable solubility when compared with crystalline form VI. Its preparation method offers good reproducibility. In addition, it exhibits valuable pharmaceutical formulation properties.

### 5. Study into the preparation and stability of pharmaceutical compositions (crystalline form, purity and content)

| 1000 capsules prescribed (dosage: 25mg) | |
|---|---|
| Form VIII | 25 mg |
| Lactose | 71.2 mg |
| Magnesium stearate | 1.3 mg |
| Stearic acid | 1.3 mg |
| Starch (Starch 1500) | 19.5 mg |
| Sodium carboxymethyl starch (CMS-Na) | 6.5 mg |

Subjected to the pharmacopoeia stability assessment method and undergoing influencing factor testing (10 day exposure): High temperature (60°C), illumination (4500 1x), high humidity (92.5%RH, 25°C); Accelerated testing (hermetically sealed for 6 months): temperature 30°C, humidity 65%RH; Long term testing (hermetically sealed for 12 months): temperature 25°C, humidity 60%RH. The assessment results demonstrate that under the above conditions neither the crystalline form, purity nor content of the product underwent any changes.

Consequently, the test results of the pharmaceutical ingredients and capsules of this product show that form VIII has a great potential in pharmaceutical production.

### Embodiment 5: Agomelatine compound of formula (II)

10g of agomelatine is added to a 100ml solution of ethyl acetate. At a temperature of 10°C, 4.6g of an aqueous solution of HCl (36%) is slowly added dropwise. Agitation is then carried out for 1 hour, followed by filtration and the resulting solid is washed twice in 10ml of ethyl acetate, then dried at a temperature of 40°C to obtain 10.2g of form II white solid; purity: 99.8%, yield: 88.7%.

### Cl elemental analysis:

Theoretically calculated value: Cl content 11.91 wt %
Measured value: Cl content 11.86 wt %

Determination of crystal water content of agomelatine compound of formula (II):
The calculated theoretical crystal water content of C₁₅H₁₇NO₂•HCl•H₂O is 6.06 wt %.

### 5.1 The Fischer method (Chinese Pharmacopoeia 2010 edition, appendix VIII M)

The product resulting from embodiment 5 was measured according to the Fischer method as mentioned above, and the crystal water content recorded was: 6.15 wt %.

### 5.2 Thermogravimetric analysis (Chinese Pharmacopoeia 2010 edition, appendix VIII Q)

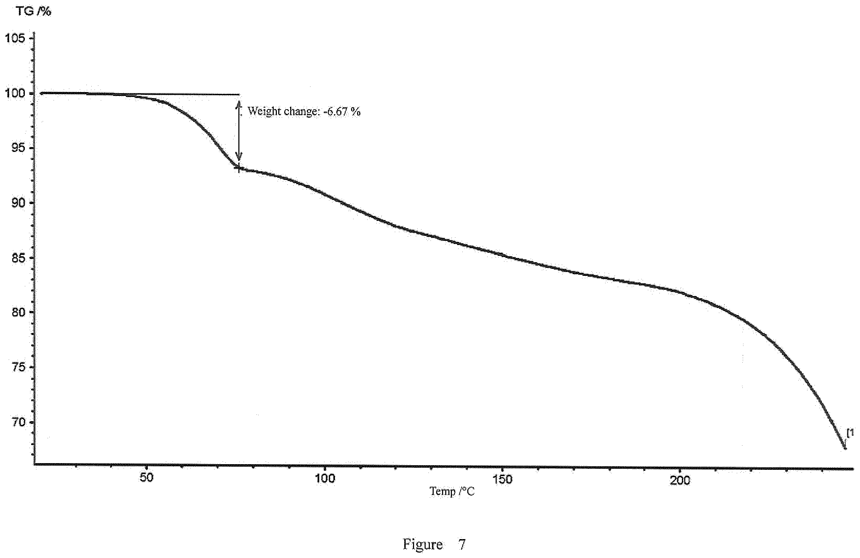

The product resulting from embodiment 5 was measured according to thermogravimetric analysis as mentioned above, and the loss of crystal water recorded was: 6.67 wt %, i.e. the crystal water content of the original product was 6.67 wt %. For TGA curve, please refer to Figure 7.

## Claims

1. Mixed crystalline form of agomelatine, its X-ray diffraction diagram having the following values for Bragg angle 2θ:
| 2θ° |
|---|
| 9.493 |
| 9.809 |
| 10.815 |
| 11.171 |
| 11.879 |
| 12.770 |
| 13.811 |
| 14.939 |
| 15.315 |
| 16.085 |
| 17.544 |
| 18.491 |
| 19.065 |
| 19.538 |
| 19.774 |
| 20.801 |
| 21.156 |
| 21.807 |
| 22.499 |
| 23.032 |
| 23.780 |
| 24.610 |
| 25.419 |
| 27.075 |
| 31.931 |
including crystals whose peak diffraction angles are within 2θ±0.2° of the above.

2. Mixed crystalline form of agomelatine, its X-ray diffraction diagram having the following values for interplanar crystal spacing d, Bragg angle 2θ and relative intensity:
| 2θ° | d (Å) | Relative intensity (I%) |
|---|---|---|
| 9.493 | 9.3085 | 12.86 |
| 9.809 | 9.0096 | 15.62 |
| 10.815 | 8.1735 | 13.10 |
| 11.171 | 7.9141 | 17.53 |
| 11.879 | 7.4439 | 64.67 |
| 12.770 | 6.9264 | 17.90 |
| 13.811 | 6.4065 | 17.10 |
| 14.939 | 5.9255 | 12.14 |
| 15.315 | 5.7808 | 10.48 |
| 16.085 | 5.5057 | 19.89 |
| 17.544 | 5.0510 | 48.47 |
| 18.491 | 4.7943 | 66.41 |
| 19.065 | 4.6512 | 24.02 |
| 19.538 | 4.5398 | 99.39 |
| 19.774 | 4.4861 | 100.00 |
| 20.801 | 4.2668 | 50.35 |
| 21.156 | 4.1961 | 30.66 |
| 21.807 | 4.0722 | 37.31 |
| 22.499 | 3.9486 | 22.63 |
| 23.032 | 3.8583 | 31.18 |
| 23.780 | 3.7387 | 39.67 |
| 24.610 | 3.6144 | 21.02 |
| 25.419 | 3.5011 | 30.30 |
| 27.075 | 3.2906 | 14.67 |
| 31.931 | 2.8004 | 14.14 |
including crystals whose peak diffraction angles are within 2θ±0.2° of the above.

3. The mixed crystalline form of agomelatine according to claim 1 or 2, **characterised by**: its DSC change-in-absorption diagram, the onset value range being 97-98°C, the endothermic peak area being no lower than 90%, with the preferable ratio being 95-99%.

4. The preparation method for the mixed crystalline form of agomelatine according to any of claims 1-3, wherein agomelatine compounds of formula (II) are dissolved in acetic acid, to which sodium acetate is then added, followed by the dropwise addition of water to this reaction mixture, which is then agitated at a temperature of 7-13°C in order to bring about crystallization, with the crystals then being separated from the solution

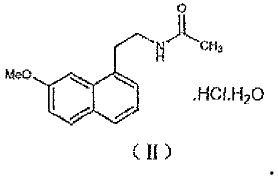

5. The preparation method according to claim 4, wherein the molar ratio of agomelatine compounds of formula (II) and sodium acetate is of the order of 1 : 1-1.5, most optimally 1 : 1-1.1.

6. The preparation method according to claim 4 or 5, wherein the ratio of volume of acetic acid to water is 1:15-30.

7. The preparation method according to any of claims 4-6, wherein when the temperature of the resulting reaction mixture reaches 12-18°C, and in particular when 15°C, water is added dropwise in order to bring about crystallization.

8. The preparation method according to any of claims 4-7, wherein water is added dropwise to the resulting reaction mixture which is then agitated at a temperature of 10°C in order to bring about crystallization.

9. The preparation method according to any of claims 4-8, wherein following the addition of the sodium acetate, the reaction mixture is heated to 40-80°C; the said solution is then left to cool on its own, and water is added dropwise in order to bring about crystallization.

10. A pharmaceutical composition, including the mixed crystalline form of agomelatine according to any of claims 1-3 and pharmaceutically acceptable adjuvants or excipients.

11. The pharmaceutical composition according to claim 10, used in the preparation of a medicament, with this medicament being used to treat diseases of the melatoninergic system.

12. The pharmaceutical composition according to claim 10, used in the preparation of a medicament, with this medicament being used to treat sleep disorders, stress, anxiety, seasonal affective disorder, severe depression, cardiovascular diseases, digestive system diseases, insomnia and fatigue brought on by jet lag, schizophrenia, phobias, or depression.

13. The pharmaceutical application of the mixed form of agomelatine according to any of claims 1-3, wherein the said pharmaceutical application relates to the treatment of diseases of the melatoninergic system.

14. The pharmaceutical application of the mixed crystalline form of agomelatine according to any of claims 1-3, wherein the said pharmaceutical application relates to the treatment of sleep disorders, stress, anxiety, seasonal affective disorder, severe depression, cardiovascular diseases, digestive system diseases, insomnia and fatigue brought on by jet lag, schizophrenia, phobias, or depression.

## Patentansprüche

1. Mischkristallform von Agomelatin, deren Röntgenbeugungsdiagramm die folgenden Bragg-Winkel-Werte 2θ aufweist:
| 2θ° |
|---|
| 9,493 |
| 9,809 |
| 10,815 |
| 11,171 |
| 11,879 |
| 12,770 |
| 13,811 |
| 14,939 |
| 15,315 |
| 16,085 |
| 17,544 |
| 18,491 |
| 19,065 |
| 19,538 |
| 19,774 |
| 20,801 |
| 21,156 |
| 21,807 |
| 22,499 |
| 23,032 |
| 23,780 |
| 24,610 |
| 25,419 |
| 27,075 |
| 31,931 |
einschließlich Kristallen, deren Röntgenbeugungspeaks innerhalb 2θ ± 0,2° der obigen Werte liegen.

2. Mischkristallform von Agomelatin, deren Röntgenbeugungsdiagramm die folgenden Werte für die Kristallnetzebenenabstände d, Bragg-Winkel 2θ und relative Intensität aufweist:
| 2θ° | d (Å) | Relative Intensität (I %) |
|---|---|---|
| 9493 | 9,3085 | 12,86 |
| 9,809 | 9,0096 | 15,62 |
| 10,815 | 8,1735 | 13,10 |
| 11,171 | 7,9141 | 17,53 |
| 11,879 | 7,4439 | 64,67 |
| 12,770 | 6,9264 | 17,90 |
| 13,811 | 6,4065 | 17,10 |
| 14,939 | 5,9255 | 12,14 |
| 15,315 | 5,7808 | 10,48 |
| 16,085 | 5,5057 | 19,89 |
| 17,544 | 5,0510 | 48,47 |
| 18,491 | 4,7943 | 66,41 |
| 19,065 | 4,6512 | 24,02 |
| 19,538 | 4,5398 | 99,39 |
| 19,774 | 4,4861 | 100,00 |
| 20,801 | 4,2668 | 50,35 |
| 21,156 | 4,1961 | 30,66 |
| 21,807 | 4,0722 | 37,31 |
| 22,499 | 3,9486 | 22,63 |
| 23,032 | 3,8583 | 31,18 |
| 23,780 | 3,7378 | 39,67 |
| 24,610 | 3,6144 | 21,02 |
| 25,419 | 3,5011 | 30,30 |
| 27,075 | 3,2906 | 14,67 |
| 31,931 | 2,8004 | 14,14 |
einschließlich Kristallen, deren Beugungswinkelpeaks innerhalb 2θ ± 0,2° der obigen Werte liegen.

3. Mischkristallform von Agomelatin nach Anspruch 1 oder 2, **gekennzeichnet durch**: ihr Absorptionsänderungs-DSC-Diagramm, dessen Onset-Wertbereich 97 - 98 °C beträgt, die Fläche des endothermen Peaks nicht geringer ist als 90 %, mit dem bevorzugten Verhältnis von 95 - 99 %.

4. Verfahren zur Herstellung der Mischkristallform von Agomelatin nach irgendeinem der Ansprüche 1 bis 3, worin Agomelatinverbindungen der Formel (II) 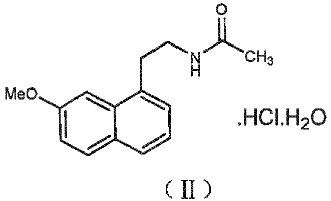 in Essigsäure gelöst werden, wonach Natriumacetat zugegeben wird, gefolgt von der tropfenweisen Zugabe von Wasser zu dieser Reaktionsmischung, die dann zur Kristallisation bei einer Temperatur von 7 - 13 °C gerührt wird, wonach die Kristalle von der Lösung getrennt werden.

5. Herstellungsverfahren nach Anspruch 4, worin das Molverhältnis von Agomelatinverbindungen der Formel (II) und Natriumacetat im Bereich von 1 : 1 - 1,5, am bevorzugtesten von 1 : 1 - 1,1 beträgt.

6. Herstellungsverfahren nach Anspruch 4 oder 5, worin das Volumenverhältnis von Essigsäure zu Wasser 1 : 15 - 30 beträgt.

7. Herstellungsverfahren nach einem der Ansprüche 4 bis 6, worin, wenn die Temperatur der sich ergebenden Reaktionsmischung 12 - 18 °C, und insbesondere 15 °C erreicht, tropfenweise Wasser zur Kristallisation zugegeben wird.

8. Herstellungsverfahren nach einem der Ansprüche 4 bis 7, worin das Wasser tropfenweise zu der gebildeten Reaktionsmischung zugegeben wird, die dann zur Kristallisation bei einer Temperatur von 10 °C gerührt wird.

9. Herstellungsverfahren nach einem der Ansprüche 4 bis 8, worin die Reaktionsmischung nach der Zugabe von Natriumacetat auf 40 - 80 °C erhitzt wird; die Lösung dann abkühlen gelassen wird und zur Kristallisation Wasser tropfenweise zugesetzt wird.

10. Pharmazeutische Zubereitung enthaltend die Mischkristallform von Agomelatin nach einem der Ansprüche 1 bis 3 und pharmazeutisch annehmbare Hilfsstoffe oder Trägermaterialien.

11. Pharmazeutische Zubereitung nach Anspruch 10 zur Verwendung bei der Herstellung eines Arzneimittels, welches Arzneimittel zur Behandlung von Erkrankungen des melatoninergischen Systems verwendet wird.

12. Pharmazeutische Zubereitung nach Anspruch 10 zur Verwendung bei der Herstellung eines Arzneimittels, welches Arzneimittel zur Behandlung von Schlafstörungen, Stress, Angst, saisonal bedingten Störungen, schwerer Depression, kardiovaskulären Erkrankungen, Erkrankungen des Verdauungssystems, Schlaflosigkeit und Müdigkeit als Folge von Jetlag, Schizophrenie, Phobien oder Depression verwendet wird.

13. Pharmazeutische Anwendung der Mischkristallform von Agomelatin nach einem der Ansprüche 1 bis 3, worin die pharmazeutische Anwendung die Behandlung von Erkrankungen des melatoninergischen Systems betrifft.

14. Pharmazeutische Anwendung der Mischkristallform von Agomelatin nach einem der Ansprüche 1 bis 3, worin die pharmazeutische Anwendung die Behandlung von Schlafstörungen, Stress, Angst, saisonal bedingten Störungen, schwerer Depression, kardiovaskulären Erkrankungen, Erkrankungen des Verdauungssystems, Schlaflosigkeit und Müdigkeit als Folge von Jetlag, Schizophrenie, Phobien oder Depression umfasst.

## Revendications

1. Forme cristalline mixte d'agomélatine, son diagramme de diffraction X présentant les valeurs suivantes pour l'angle de Bragg 2θ :
| 2θ° |
|---|
| 9,493 |
| 9,809 |
| 10,815 |
| 11,171 |
| 11,879 |
| 12,770 |
| 13,811 |
| 14,939 |
| 15,315 |
| 16,085 |
| 17,544 |
| 18,491 |
| 19,065 |
| 19,538 |
| 19,774 |
| 20,801 |
| 21,156 |
| 21,807 |
| 22,499 |
| 23,032 |
| 23,780 |
| 24,610 |
| 25,419 |
| 27,075 |
| 31,931 |
y compris les cristaux dont les angles de diffraction de pic se trouvent à 2θ ± 0,2° de ceux ci-dessus.

2. Forme cristalline mixte d'agomélatine, son diagramme de diffraction X présentant les valeurs suivantes pour la distance inter-réticulaire d des cristaux, l'angle de Bragg 2θ et l'intensité relative :
| 2θ° | d(Å) | Intensité relative (I en %) |
|---|---|---|
| 9,493 | 9,3085 | 12,86 |
| 9,809 | 9,0096 | 15,62 |
| 10,815 | 8,1735 | 13,10 |
| 11,171 | 7,9141 | 17,53 |
| 11,879 | 7,4439 | 64,67 |
| 12,770 | 6,9264 | 17,90 |
| 13,811 | 6,4065 | 17,10 |
| 14,939 | 5,9255 | 12,14 |
| 15,315 | 5,7808 | 10,48 |
| 16,085 | 5,5057 | 19,89 |
| 17,544 | 5,0510 | 48,47 |
| 18,491 | 4,7943 | 66,41 |
| 19,065 | 4,6512 | 24,02 |
| 19,538 | 4,5398 | 99,39 |
| 19,774 | 4,4861 | 100,00 |
| 20,801 | 4,2668 | 50,35 |
| 21,156 | 4,1961 | 30,66 |
| 21,807 | 4,0722 | 37,31 |
| 22,499 | 3,9486 | 22,63 |
| 23,032 | 3,8583 | 31,18 |
| 23,780 | 3,7387 | 39,67 |
| 24,610 | 3,6144 | 21,02 |
| 25,419 | 3,5011 | 30,30 |
| 27,075 | 3,2906 | 14,67 |
| 31,931 | 2,8004 | 14,14 |
y compris les cristaux dont les angles de diffraction de pic se trouvent à 2θ ± 0,2° de ceux ci-dessus.

3. Forme cristalline mixte d'agomélatine selon la revendication 1 ou 2, **caractérisée par** : son diagramme de changement d'absorption par DSC, la plage de valeurs de départ étant de 97 à 98 °C, la surface du pic endothermique n'étant pas inférieure à 90 %, le rapport préféré étant de 95 à 99 %.

4. Procédé de préparation de la forme cristalline mixte d'agomélatine selon l'une quelconque des revendications 1 à 3, dans lequel les composés d'agomélatine de formule (II) sont dissous dans de l'acide acétique, auquel de l'acétate de sodium est ensuite ajouté, puis de l'eau est ajoutée goutte-à-goutte à ce mélange réactionnel, qui est ensuite agité à une température de 7 à 13 °C afin de provoquer une cristallisation, les cristaux étant ensuite séparés de la solution

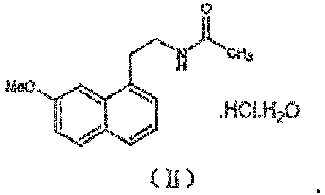

5. Procédé de préparation selon la revendication 4, dans lequel le rapport molaire des composés d'agomélatine de formule (II) et de l'acétate de sodium est de l'ordre de 1/1 à 1/1,5, de manière la plus optimale de 1/1 à 1/1,1.

6. Procédé de préparation selon la revendication 4 ou 5, dans lequel le rapport du volume d'acide acétique par rapport à celui de l'eau est de 1/15 à 1/30.

7. Procédé de préparation selon l'une quelconque des revendications 4 à 6, dans lequel, quand la température du mélange réactionnel résultant atteint 12 à 18 °C, et en particulier 15 °C, de l'eau est ajoutée goutte-à-goutte afin de provoquer une cristallisation.

8. Procédé de préparation selon l'une quelconque des revendications 4 à 7, dans lequel de l'eau est ajoutée goutte-à-goutte au mélange réactionnel résultant qui est ensuite agité à une température de 10 °C afin de provoquer une cristallisation.

9. Procédé de préparation selon l'une quelconque des revendications 4 à 8, dans lequel, après l'ajout de l'acétate de sodium, le mélange réactionnel est chauffé à une température de 40 à 80 °C ; ladite solution est ensuite laissée à refroidir par elle-même, et de l'eau est ajoutée goutte-à-goutte afin de provoquer une cristallisation.

10. Composition pharmaceutique, comprenant la forme cristalline mixte d'agomélatine selon l'une quelconque des revendications 1 à 3 et des adjuvants ou excipients pharmaceutiquement acceptables.

11. Composition pharmaceutique selon la revendication 10, utilisée dans la préparation d'un médicament, ce médicament étant utilisé pour traiter les maladies du système mélatoninergique.

12. Composition pharmaceutique selon la revendication 10, utilisée dans la préparation d'un médicament, ce médicament étant utilisé pour traiter les troubles du sommeil, le stress, l'anxiété, le trouble affectif saisonnier, la dépression majeure, les maladies cardio-vasculaires, les maladies du système digestif, l'insomnie et la fatigue provoquées par le décalage horaire, la schizophrénie, les phobies, ou la dépression.

13. Application pharmaceutique de la forme mixte d'agomélatine selon l'une quelconque des revendications 1 à 3, dans laquelle ladite application pharmaceutique concerne le traitement des maladies du système mélatoninergique.

14. Application pharmaceutique de la forme cristalline mixte d'agomélatine selon l'une quelconque des revendications 1 à 3, dans laquelle ladite application pharmaceutique concerne le traitement des troubles du sommeil, du stress, de l'anxiété, du trouble affectif saisonnier, de la dépression majeure, des maladies cardio-vasculaires, des maladies du système digestif, de l'insomnie et de la fatigue provoquées par le décalage horaire, de la schizophrénie, des phobies, ou de la dépression.
